# EUROPÄISCHE PATENTANMELDUNG

(11) **EP 2 301 436 A1**
(43) Veröffentlichungstag der Anmeldung: **30.03.2011**
(21) Anmeldenummer: 09171070.7
(22) Anmeldetag: 23.09.2009
(51) Int. Cl.: A61B 5/103

(54) **Vorrichtung und Verfahren zur Messung der elastischen und viskoelastischen Verformbarkeit von Haut**

(71) Anmelder: Courage + Khazaka electronic GmbH, 50829 Köln (DE)
(72) Erfinder: Khazaka, Gabriel, 50859 Köln (DE)
(74) Vertreter: von Kreisler Selting Werner

(57) **Zusammenfassung**

Bei einer Vorrichtung zur Messung der elastischen und viskoelastischen Verformbarkeit von Haut mit einer Messsonde (1), die einen die Hautoberfläche (2) mit einem pneumatischen Druck beaufschlagenden Messsondenkanal (3) aufweist, mit dem eine Wegmessung des Deformationsweges der Hautoberfläche durchführbar ist, wobei zur Wegmessung eine Lichtschranke (4) vorgesehen ist, deren Messstrecke (7) im Bereich von und parallel zu einer auf die Haut aufsetzbaren Öffnung (5, 23) des Messsondenkanals (3) verläuft, und mit einer elektronischen Auswerteeinrichtung, ist vorgesehen, dass die Messsonde (1) an der Öffnung (5, 23) des Messsondenkanals (3) eine die Öffnung (5, 23) umgebende Saugeinrichtung (56) aufweist.

## Beschreibung

Die Erfindung betrifft eine Vorrichtung und ein Verfahren zur Messung der elastischen und viskoelastischen Verformbarkeit von Haut nach dem Oberbegriff der Ansprüche 1, 8 und 13.

Bislang für solche Zwecke verwendeten, aus der EP 0 362 616 A bekannten Vorrichtungen weisen eine Messsonde, die eine Wegmesseinrichtung zur Messung des Deformationswegs der Hautoberfläche aufweisen, und eine elektronische Auswerteeinrichtung auf. Die Messsonde weist ferner einen Messsondenkanal auf, der die Hautoberfläche mit einem pneumatischen Druck beaufschlagt. Zur Wegmessung ist im Bereich des Messsondenkanals eine Lichtschranke angeordnet, deren Messstrecke quer zum Messsondenkanal bis zur Ebene seiner auf die Haut aufsetzbaren Öffnung verläuft.

Bei diesen bisher bekannten Vorrichtungen besteht der Nachteil, dass die Messsonde möglichst exakt orthogonal auf der zu messenden Hautoberfläche positioniert werden müssen, um eine unverfälschte reproduzierbare Messung vornehmen zu können.

Der Erfindung liegt demzufolge die Aufgabe zugrunde, eine Vorrichtung und ein Verfahren der eingangs beschriebenen Art anzugeben, mit denen präzisere Messungen vorgenommen werden können und besser reproduzierbare Messergebnisse erzielbar sind.

Zur Lösung dieser Aufgabe dienen die Merkmale der Ansprüche 1, 8 und 13.

Die Erfindung sieht in vorteilhafter Weise vor, dass die Messsonde an der auf die Haut aufsetzbaren Öffnung des Messsondenkanals eine die Öffnung umgebende Saugeinrichtung aufweist.

Dies hat den Vorteil, dass die Saugeinrichtung die Haut an die Messsonde ansaugt und somit den Messsondenkanal in Bezug auf die zu messende Hautoberfläche exakt orthogonal positioniert. Es wird sowohl ein Verrutschen der Messsonde als auch eine von der orthogonalen abweichenden Lage der Messsonde in Bezug auf die zu messende Hautoberfläche während der Messung verhindert. Somit ist eine präzise Messung möglich und das Messergebnis ist in einem höheren Umfang reproduzierbar.

Die Messstrecke kann bis zur Ebene der auf die Haut aufsetzbaren Öffnung des Messsondenkanals verlaufen. Dies hat den Vorteil, dass sehr genaue Messungen möglich sind.

Das Saugelement kann ringförmig ausgebildet sein.

Die Saugeinrichtung kann auf der der Haut zugewandten Seite mit einer Vielzahl von vorzugsweise gleichmäßig beabstandeten Saugöffnungen versehen sein.

Die Haut wird nicht in die vorzugsweise kleinen, mit Unterdruck beaufschlagten Saugöffnungen hineingezogen. Dies hat den Vorteil, dass die Hautmessung durch die Saugeinrichtung nicht verfälscht wird und die Haut im Umfangsbereich zu dem Messbereich in definierbarer und reproduzierbarer Weise gehalten wird.

Die Saugöffnungen können in der Draufsicht rund oder schlitzförmig sein.

Die Saugeinrichtung kann auch aus zwei halbkreisförmigen Elementen gebildet sein und die Messtrecke kann zwischen den sich zu einem Saugring ergänzenden halbkreisförmigen Elemente verlaufen.

Die Saugeinrichtung kann aus einem flexiblen Schlauchmaterial gebildet sein, z. B. vorzugsweise aus Silikon. Auch andere Materialien sind verwendbar, wenn eine gute Haftreibung gegenüber der Haut erzielbar ist.

Die Saugeinrichtung kann mehrere im Wesentlichen ringförmige Elemente aufweisen, die konzentrisch zueinander angeordnet sind, wobei zwischen den konzentrisch angeordneten Elementen jeweils eine ringförmige Saugöffnung angeordnet sein kann.

Die Messsonde ist vorzugsweise zumindest teilweise stabförmig ausgebildet.

Die Messsonde weist vorzugsweise an ihrem freien Ende einen Messkopf auf. Der Messkopf kann eine Stirnseite und zumindest eine Seitenfläche aufweisen. Die Seitenfläche kann im Wesentlichen als Mantelfläche im Sinne einer Ummantelung ausgebildet sein. Die Stirnseite an dem freien distalen Ende ist vorzugsweise konvex geformt. Die Seitenfläche verläuft im Wesentlichen orthogonal zur Stirnseite.

Die auf die Haut aufsetzbare Öffnung kann an der Stirnseite des Messkopfs angeordnet sein.

Alternativ kann an der Seitenfläche des Messkopfs eine auf die Haut aufsetzbare Öffnung angeordnet sein.

Diese Ausführungsform sieht in vorteilhafter Weise vor, dass die auf die Haut aufsetzbare Öffnung der Messsonde an der Seitenfläche des Messkopfs angeordnet ist.

Dies hat den Vorteil, dass Messungen auch in Körperöffnungen z. B. an Wandungen der Körperöffnungen durchgeführt werden können. In Körperöffnungen besteht bei Messsonden mit einer auf die Haut aufsetzbaren Öffnung an der Stirnseite das Problem, dass diese nicht richtig in Bezug auf die zu messende Hautoberfläche positioniert werden können. Somit sind Messungen in insbesondere engen Körperöffnungen mit Messsonden mit einer auf die Haut aufsetzbaren Öffnung an der Stirnseite häufig nicht möglich.

Der Messkopf kann auswechselbar sein. Ferner kann der Messkopf sterilisierbar sein.

Die Messsonde ist vorzugsweise an der Stelle der auf die Haut aufsetzbaren Öffnung abgeflacht.

Die Auswerteeinrichtung kann das Messsignal verwerfen, wenn die Saugeinrichtung keinen Unterdruck auf der Haut erzeugt.

Der Messkopf der Messsonde kann zumindest teilweise aus einem Prismenkörper bestehen, durch den die Messstrecke verläuft. Der Prismenkörper kann aus Glas oder aus Kunststoff bestehen.

Der Lichtsender und der Lichtempfänger der Lichtschranke können auf gegenüberliegenden Seiten der auf die Haut aufsetzbaren Öffnung der Messsonde integriert sein und der Lichtstrahl der Lichtschranke kann über Spiegel zweifach umgelenkt sein.

Vorzugsweise weist die gesendete bzw. empfangene Lichtstrahlung eine Wellenlänge im Bereich zwischen 490 und 575 nm auf.

Es versteht sich, dass auch andere Wellenlängenbereiche verwendbar sind. Die emittierte Strahlung kann auch moduliert sein.

Der Lichtsender kann aus einer Leuchtdiode, z. B. eine Infrarotleuchtdiode und der Lichtempfänger aus einer Photodiode, z. B. einer Infrarotphotodiode bestehen.

Die auf die Haut aufsetzbare Öffnung des Messsondenkanals kann einen Durchmesser zwischen ca. 2 und ca. 12 mm aufweisen.

Die Messsonde kann mit einem von einer Feder gegen die Körperoberfläche vorgespannten Messkopf versehen sein.

Eine pneumatische Druckversorgungseinrichtung, bestehend aus einer Pumpe, einem Druckspeicher und einem Drosselventil, kann den Druck im Messsondenkanal erzeugen, wobei die Steuerung der Pumpe, des Druckspeicherdrucks und der Drosselventilstellung über einen Mikroprozessor erfolgt, der auch die elektronische Schaltung der Messsonde ansteuert.

Der Druck in dem Messsondenkanal kann ein Unterdruck sein.

Der Druck in dem Messsondenkanal kann alternativ auch ein Überdruck sein, wobei ein in dem Messsondenkanal frei bewegliches, mit dem Überdruck beaufschlagtes Kolbenelement in die Messstrecke der Lichtschranke mit seinem druckbeaufschlagten Ende hineinragen kann.

Bei einem Verfahren zur Messung der elastischen und viskoelastischen Verformbarkeit von Haut durch Beaufschlagung der Hautoberfläche mit einem Druck über eine Messsonde und durch Messen der Hautdeformation auf Grund der Druckbelastung über eine Deformationswegmessung, wobei der Druck pneumatisch auf die zu messende Hautoberfläche über eine Öffnung eines Messsondenkanal aufgebracht wird, und die Hautdeformation mit Hilfe einer in dem druckbeaufschlagten Messsondenkanal angeordneten Lichtschranke gemessen wird, deren Änderung der Lichtintensität als Maß für die Deformation der Hautoberfläche verwendet wird, kann die Haut im Umfeld der Öffnung des Messsondenkanals angesaugt werden.

Die Hautoberfläche kann mit einem Unterdruck beaufschlagt werden und die von der Photodiode gemessene Lichtintensität kann durch die in die Messstrecke angesaugte Haut der Hautoberfläche verringert werden.

Die Hautoberfläche kann über einen frei im Messsondenkanal beweglichen Kolbenelement mit einem Überdruck beaufschlagt werden und die gemessene Lichtintensität der Lichtschranke kann durch die Lage des Kolbenelementes beeinflusst werden. Die Kombination der Beaufschlagung der Haut mit dem Kolbenelement mit der Ansaugung über die die Öffnung mit dem Stößel umgebende Saugeinrichtung eröffnet zusätzliche Analysemöglichkeiten.

Die Messsonde kann mit bereits anliegendem Unter- bzw. Überdruck in dem Messsondenkanal auf die Hautoberfläche aufgesetzt werden.

Nach dem Aufsetzen der Messsonde kann der Unterdruck bzw. Überdruck in dem Messsondenkanal von dem Umgebungsdruck auf einen Sollwert erhöht werden.

Nach dem Aufsetzen der Messsonde kann eine Wechselbelastung auf die Hautoberfläche ausgeübt werden, indem der Unter- bzw. Überdruck zwischen einem ersten und einem zweiten Grenzwert mehrfach verändert wird.

Im Folgenden werden unter Bezugnahme auf die Zeichnungen Ausführungsbeispiele der Erfindung näher erläutert.

Es zeigen schematisch:
- Fig. 1: eine Messsonde zur Beaufschlagung der Hautoberfläche mit einem Unterdruck,
- Fig. 2: eine Messsonde zur Beaufschlagung der Hautoberfläche mit einem Überdruck,
- Fig. 3a: eine Unteransicht der Messsonde,
- Fig. 3b: eine Unteransicht der Messsonde mit einer alternativen Saugeinrichtung,
- Fig. 3c: eine Unteransicht der Messsonde mit einer weiteren Saugein- richtung.
- Fig. 4: eine Messsonde mit seitlicher auf die Haut aufsetzbaren Öff- nung,
- Fig. 5: eine Schnittansicht der Messsonde aus Figur 4,
- Fig. 6: eine Unteransicht der Messsonde aus Figur 4

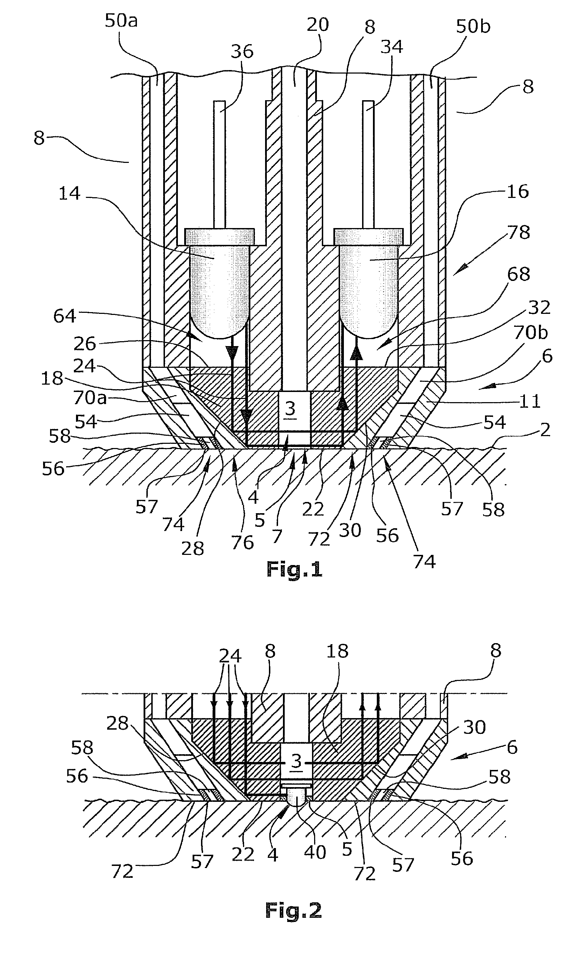

Figur 1 zeigt eine Messsonde mit einer Lichtschranke 4. Die Messsonde weist ein Gehäuse 8 und ein Messkopf 6 aufweist. In dem Gehäuse 8 sind Kanäle 20, 50a und 50b angeordnet, die an eine pneumatische Druckleitung anschließbar sind. Ferner sind in dem Gehäuse 8 Lichtleitkanäle 64, 68 der Lichtschranke 4 angeordnet. In dem Lichtleitkanal 64 ist eine Leuchtdiode 14 angeordnet, die vorzugsweise grünes Licht sendet. In dem Lichtleitkanal 68 ist eine Photodiode 16 angeordnet, die vorzugsweise grünes Licht empfangen kann. Auch andere Wellenlängenbereiche, die mit Leuchtdioden und Fotodioden anwendbar sind, können verwendet werden. Die Strahlung kann moduliert sein. An dem Gehäuse 8 ist ein Messkopf 6 befestigt. Der Messkopf 6 kann beispielsweise über eine Bajonettverbindung mit dem Gehäuse 8 verbunden werden. Der Messkopf 6 weist ein Gehäuse 11 und einen Prismenkörper 18 als Bestandteil der Lichtschranke 4 auf. In dem Gehäuse 11 sind zwei Luftkanäle 70a und 70b angeordnet, wobei der Luftkanal 70a mit dem Kanal 50a des Gehäuses 8 und der Luftkanal 70b mit dem Kanal 50b des Gehäuses 8 verbunden ist. Die Luftkanäle 70a und 70b enden in einem ringförmigen Hohlraum 54, der ebenfalls in dem Gehäuse 11 des Messkopf 6 angeordnet ist. Der Hohlraum weist eine ringförmige auf die Haut aufsetzbare Öffnung 74 auf.

In dem Prismenkörper 18 ist ein Messsondenkanal 3 angeordnet, der mit dem Kanal 20 verbunden ist. Der Messsondenkanal 3 weist eine auf die Haut aufsetzbare Öffnung 5 auf, die bündig mit einer Auflagefläche 22 des Prismenkörpers 18 abschließt. Die Öffnung 5 verläuft orthogonal zur Längsrichtung des Messsondenkanals 3. Das Gehäuse 11 weist ebenfalls eine Auflagefläche 72 auf, die bündig mit der Auflagefläche 22 des Prismenkörpers 18 und mit der Öffnung 5 des Messsondenkanals 3 abschließt.

Die Lichtschranke 4 weist also die Leuchtdiode 14, die Photodiode 16, die Lichtleitkanäle 64,68, den Prismenköper 18 und die Messstrecke 7 in dem Messsondenkanal 3 auf.

Die Öffnung 74 des ringförmigen Hohlraums 54 schließt ebenfalls bündig mit der Auflagefläche 72 des Gehäuses 11 ab. In dem ringförmigen Hohlraum 54 ist eine ringförmige Saugeinrichtung 56 angeordnet, die an die Breite des ringförmigen Hohlraums 54 angepasst ist. Die Saugeinrichtung 56 weist eine Auflagefläche 57 auf, die bündig mit der Öffnung 74 des ringförmigen Hohlraums 54 abschließt. Ebenso schließt die Auflagefläche 57 der Saugeinrichtung 56 bündig mit der Auflagefläche 72 des Gehäuses 11, der Auflagefläche 22 des Prismenkörpers 18 und mit der Öffnung 5 der Messsondenkanal 3 ab. Die Saugeinrichtung 56 besteht vorzugsweise aus Silikon. Die Saugeinrichtung 56 weist eine Vielzahl von vorzugsweise gleichmäßig beabstandeten Saugöffnungen 58 auf. Die Saugöffnungen 58 verlaufen im Wesentlichen parallel zu der Längsrichtung des Messsondenkanals 3. Die Saugeinrichtung 56 ist derart an die Breite des Hohlraums 54 angepasst, dass die Luft lediglich durch die Saugöffnungen 58 der Saugeinrichtung 56 von außen in den Hohlraum 54 eindringen kann.

Die als Lichtsender und Lichtempfänger dienenden Dioden 14, 16 verlaufen parallel zu dem Kanal 20 bzw. 3, wobei die Lichtstrahlen 24 zunächst parallel zu dem Messsondenkanal 3 in dem Lichtleitkanal 64 verlaufen. Die Lichtstrahlen 24 treten am Ende des Lichtleitkanals 64 durch eine erste geschliffene Fläche 26 des Prismenkörpers 18 in den Prismenkörper 18 ein. Die Fläche 26 des Prismenkörpers 18 verläuft rechtwinklig zu dem Messsondenkanal 3. Der Prismenkörper 18 weist eine unter einem Winkel von 45° zu den Lichtstrahlen 24 verlaufende erste Spiegelfläche 28 auf, die die Lichtstrahlen 24 um 90° umlenkt, so dass sie den Messsondenkanal 3 unter einem Winkel von 90° kreuzen. Der Bereich, wo die Lichtstrahlen 24 den Messsondenkanal 3 durchqueren wird Messstrecke 7 genannt. Nachdem die Lichtstrahlen 24 den Messsondenkanal 3 durchquert haben, treffen die Lichtstrahlen 24 auf eine unter einem Winkel von 45° zu den Lichtstrahlen 24 verlaufende zweite Spiegelfläche 30, die die Lichtstrahlen um 90° umlenkt, und auf die Diode 16 richtet. Die Lichtstrahlen 24 durchqueren nach dem Umlenken an der zweiten Spiegelfläche 30 den Prismenkörper 18 und verlassen den Prismenkörper 18 an einer quer zu der Längsachse des Messsondenkanal 3 verlaufenden zweiten geschliffenen Fläche 32 des Prismenkörpers 18. Die Spiegelflächen 28, 30 verlaufen bis zur Auflagefläche 22 des Prismenkörpers 18 und der ermöglichen somit die Abtastung des Messsondenkanals 3 bis unmittelbar in die Ebene der Auflagefläche 22. Die Spiegelflächen 28, 30 ermöglichen dabei die Umlenkung aller von der Diode 14 ausgehenden Lichtstrahlen 24, die parallel zu dem Messsondenkanal verlaufen.

Die Dioden 14, 16 sind mit Anschlüssen 34, 36 versehen, die mit einer elektronischen Schaltung zur Steuerung des Lichtsenders und zur Verstärkung des Messsignals des Lichtempfängers verbunden sind, die an der Messsonde 1 befestigt sind.

Der in dem Gehäuse 8 angeordnete Kanal 20 und damit der Messsondenkanal 3 werden über eine pneumatische Druckleitung mit einen Unterdruck beaufschlagt, wodurch die Hautoberfläche 2 an der Öffnung 5 je nach Flexibilität der Hautoberfläche 2 unterschiedlich weit in dem Messsondenkanal 3 hineingesogen wird. Dadurch ergibt sich eine Schwächung der von dem Lichtsender auf den Lichtempfänger übertragenen Lichtintensität, die als Messsignal für die maximale Höhe der Hautwölbung und damit für die Elastizität der Haut verwendet wird. Die Höhe der Hautwölbung wird als Deformationsweg der Hautoberfläche verstanden.

Auch werden die Kanäle 50a, 50b und damit die Kanäle 70a und 70b und somit der Hohlraum 54 über eine pneumatische Druckleitung mit Unterdruck beaufschlagt. Der Unterdruck, der in dem Hohlraum 54 anliegt, liegt ebenfalls an den Saugöffnungen 58 der Saugeinrichtung 56 an. An den Saugöffnungen 58 der Saugeinrichtung 56 wird ebenfalls die Haut angesaugt. Da jedoch die Saugöffnung 58 sehr klein sind, wird die Haut nicht bzw. nur minimal in die Saugöffnung hineingesaugt. Die Haut wird über die Saugeinrichtung 56 lediglich leicht angesaugt. Durch das Ansaugen der Haut mittels der Saugeinrichtung 56, kann die Messsonde auf der Haut genau ausgerichtet werden. Ein Verrutschen der Messsonde 1 ist nicht mehr möglich. Auch ist ein Kippen der Messsonde 1 bezogen auf die zu messende Hautoberfläche 2 nicht mehr möglich. Der Messsondenkanal 3 kann somit bezogen auf die Hautoberfläche 2 genau ausgerichtet werden. Die Längsrichtung des Messsondenkanals 3 kann somit genau im rechten Winkel zur zu messenden Hautoberfläche 2 ausgerichtet werden. Somit ist eine exakte Messung der Hautelastizität möglich. Auch ist das Messergebnis ist dadurch in verbesserten Umfang reproduzierbar.

Figur 2 zeigt ein anderes Ausführungsbeispiel, bei dem der Kanal 20 und der Messsondenkanal 3 mit einem Druck beaufschlagt werden, wodurch ein bewegliches Kolbenelement 40 in die Hautoberfläche 2 eingedrückt wird. Das druckbeauftragte Ende des beweglichen Kolbenelementes 40 reicht in die Messstrecke 7 der Lichtschranke 4 hinein und beeinflusst somit je nach Eindellung der Hautoberfläche 2 die von dem Lichtsensor auf dem Lichtempfänger übertragene Lichtintensität. Das auf die Hautoberfläche 2 drückende Ende des Kolbenelements 40 kann gradzylindrisch, kalottenförmig oder spitz auslaufend gestaltet sein. Die Kanäle 50a, 50b und somit die Kanäle 70a, 70b und der Hohlraum 54 werden wie beim ersten Ausführungsbeispiel mit Unterdruck beaufschlagt. Somit werden auch die Saugöffnungen 58 der Saugeinrichtung 56 mit Unterdruck beaufschlagt.

Der Messkopf 6 kann als auswechselbares Teil ausgestaltet sein, derart dass unterschiedliche Kolbenelementformen, z. B. spitz, rund oder flach, einsetzbar sind.

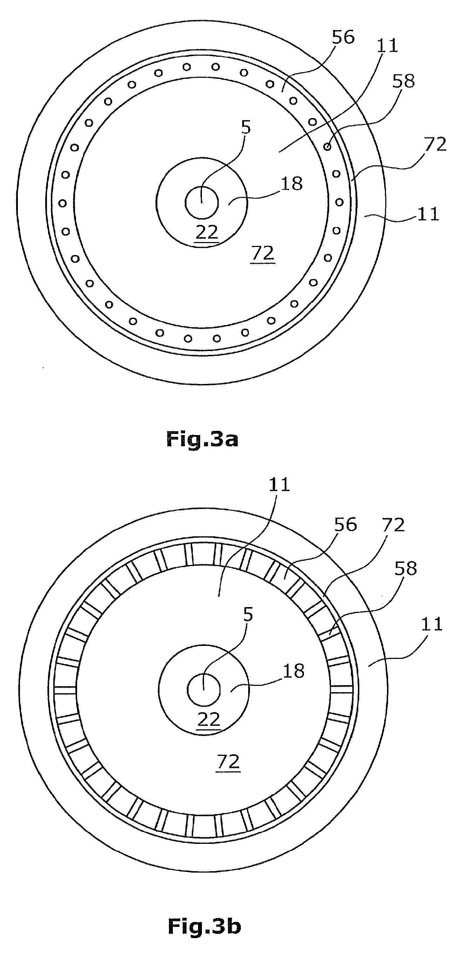

Figur 3a zeigt das Ausführungsbeispiel aus Figur 1 in der Unteransicht. In Figur 3a ist deutlich zu erkennen, dass die Saugeinrichtung ringförmig ausgebildet ist. Alternative könnte die Saugeinrichtung auch aus zwei halbkreisförmigen Elementen bestehen. Der Messsondenkanal 3 ist vorzugsweise innerhalb der Saugeinrichtung 56 angeordnet.

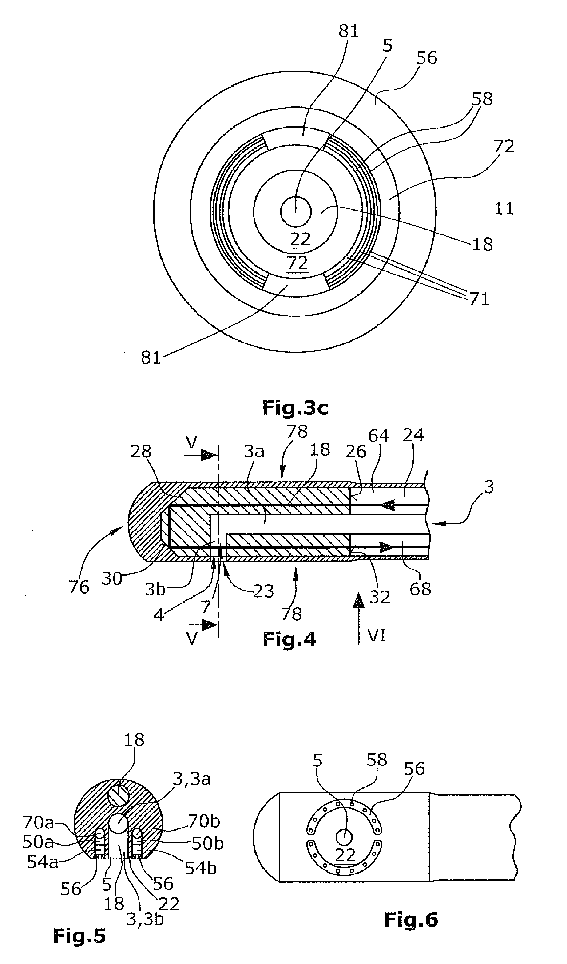

Die Figuren 3b und 3c zeigen alternative Saugeinrichtungen 56.

Die Saugeinrichtung 56 in Figur 3b weist die Saugöffnungen 58 bildende Schlitze auf. Die Saugeinrichtung 56 in Figur 3c besteht aus zwei konzentrisch zueinander angeordneten und voneinander beanstandeten, im Wesentlichen ringförmigen Elementen 71. Die Elemente 71 sind über Stege 81 miteinander verbunden. Sowohl die inneren als auch die äußeren im Wesentlichen ringförmigen Elemente 71 können an dem Gehäuse 11 anklebbar sein. Die Saugöffnung 58 ist somit als im Wesentlichen ringförmige Schlitze ausgebildet.

Figur 4 zeigt ein weiteres Ausführungsbeispiel. Das Ausführungsbeispiel unterscheidet sich von dem Ausführungsbeispiel gemäß Fig. 1 dadurch, dass die auf die Haut aufsetzbare Öffnung 23 des Messsondenkanals 3 an der Seite der Messsonde 1 angeordnet. Der Messkopf 6 weist eine Stirnseite 76 auf. Die Stirnseite 76 kann konvex geformt sein. Ferner weist der Messkopf 6 eine Seitenfläche 78 auf, die im Wesentlichen zylindrisch geformt ist. Die Öffnung 23 ist an der Seitenfläche 78 angeordnet. An der Stelle der Öffnung 23 ist die Seitenfläche 78 abgeflacht. Die Lichtstrahlen 24 treten zunächst an der Fläche 26 des Prismenkörpers 18 in den Prismenkörper 18 ein und werden dann an der ersten Spiegelfläche 28, die unter einem Winkel von 45° zu den Lichtstrahlen 24 verläuft, um 90° umgelenkt. Diese umgelenkten Lichtstrahlen 24 werden dann noch einmal an der zweiten Spiegelfläche 30, die unter einem Winkel von 45° zu den bereits umgelenkten Lichtstrahlen 24 verläuft, um 90° umgelenkt. Diese zweimal umgelenkten Lichtstrahlen 24 durchqueren dann den Messsondenkanal 3 um dann wieder in den Prismenkörper 18 einzutreten. Diese Lichtstrahlen 24 werden danach an die Diode 16 geleitet.

Der Messsondenkanal 3 weist einen ersten Abschnitt 3a auf, der in Richtung der Längsachse der Messsonde 1 verläuft. Ferner weist der Messsondenkanal 3 einen zweiten Messsondenkanal-Abschnitt 3b auf, der quer zu der Längsachse der Messsonde 1 verläuft. Dieser zweite Messsondenkanal-Abschnitt 3b endet in der Öffnung 23.

Bei dieser Ausführungsform wird die Hautelastizität auf die gleiche Art und Weise gemessen wie bei dem ersten Ausführungsbeispiel.

Dieses Ausführungsbeispiel kann beispielsweise in der Gynäkologie verwendet werden, wobei die Messsonde in Körperöffnungen eingeführt werden kann. Ferner ist der Messkopf 6 abnehmbar. Dieser abgenommene Messkopf 6 kann sterilisierbar ausgeführt sein. Alternativ könnte er für eine Einmalverwendung als Wegwerfteil ausgeführt sein.

Das Ausführungsbeispiel aus Figur 4 kann alternativ derart ausgeführt sein, dass der Prismenkörper 18 bis zur Ebene der Haut aufsetzbaren Öffnung 23 des Messsondenkanals 3 verläuft und somit auf der Hautoberfläche aufliegt. Die Messstrecke 7 kann somit bis zur Ebene der auf die Haut aufsetzbaren Öffnung 23 verlaufen.

In Figur 5 ist das Ausführungsbeispiel aus Figur 4 im Schnitt dargestellt. In Figur 5 ist die Saugeinrichtung 56 dargestellt. Die Saugeinrichtung 56 besteht aus zwei halbkreisförmigen Elementen, die sich zu einem Saugring ergänzen. Die Saugeinrichtung 56 ist über die Hohlräume 54a und 54b und die Kanäle 50a, 50b und 70a, 70b ebenfalls mit einer pneumatischen Druckleitung verbunden. Über diese Druckleitung kann wie bei dem bereits beschriebenen Ausführungsbeispiel Unterdruck an die Saugeinrichtung 56 und somit an die Saugöffnung 58 angelegt werden.

Das Ausführungsbeispiel aus Figur 4 kann anders als dargestellt alternativ ohne Saugeinrichtung ausgeführt sein. Dieses alternative Ausführungsbeispiel sähe im Schnitt genauso aus wie das Ausführungsbeispiel aus Figur 4.

In Figur 6 ist das Ausführungsbeispiel aus Figur 5 in der Unteransicht dargestellt. Hier sind die halbkreisförmigen Saugeinrichtungen 56 und die Öffnung 23 zu erkennen.

## Patentansprüche

1. Vorrichtung zur Messung der elastischen und viskoelastischen Verformbarkeit von Haut mit
- einer Messsonde (1), die einen die Hautoberfläche (2) mit einem pneumatischen Druck beaufschlagenden Messsondenkanal (3) aufweist, mit dem eine Wegmessung des Deformationsweges der Hautoberfläche durchführbar ist, wobei zur Wegmessung eine Lichtschranke (4) vorgesehen ist, deren Messstrecke (7) im Bereich von und parallel zu einer auf die Haut aufsetzbaren Öffnung (5, 23) des Messsondenkanals (3) verläuft, und
- mit einer elektronischen Auswerteeinrichtung,
**dadurch gekennzeichnet,**
**dass** die Messsonde (1) an der Öffnung (5, 23) des Messsondenkanals (3) eine die Öffnung (5, 23) umgebende Saugeinrichtung (56) aufweist.

2. Vorrichtung nach Anspruch 1, **dadurch gekennzeichnet, dass** die Messstrecke (7) bis zur Ebene der auf die Haut aufsetzbaren Öffnung (5, 23) des Messsondenkanals (3) verläuft.

3. Vorrichtung nach einem der Ansprüche 1 oder 2, **dadurch gekennzeichnet, dass** die Saugeinrichtung (56) auf der der Haut zugewandten Seite mit einer Vielzahl von vorzugsweise gleichmäßig beabstandeten Saugöffnungen (56) versehen ist.

4. Vorrichtung nach einem der Ansprüche 1 bis 3, **dadurch gekennzeichnet, dass** die Saugeinrichtung (56) aus zwei halbkreisförmigen Elementen gebildet ist und dass die Messtrecke (7) zwischen den sich zu einem Saugring ergänzenden halbkreisförmigen Elemente verläuft.

5. Vorrichtung nach einem der Ansprüche 1 bis 4, **dadurch gekennzeichnet, dass** die Saugeinrichtung (56) aus flexiblem Material gebildet ist.

6. Vorrichtung nach einem der Ansprüche 1 bis 5, **dadurch gekennzeichnet, dass** die Saugeinrichtung (56) mehrere im Wesentlichen ringförmige Elemente (71) aufweist, die konzentrisch zueinander angeordnet sind, wobei zwischen den konzentrisch angeordneten Elementen (71) jeweils eine im Wesentlichen ringförmige Saugöffnung (56) angeordnet ist.

7. Vorrichtung nach einem der Ansprüche 1 bis 6, **dadurch gekennzeichnet, dass** die Messsonde (1) an ihrem freien Ende einen Messkopf (6) aufweist und dass die auf die Haut aufsetzbare Öffnung (5, 23) an der Stirnseite (76) oder an der Seitenfläche (78) des Messkopfs (6) angeordnet ist.

8. Vorrichtung zur Messung der elastischen und viskoelastischen Verformbarkeit von Haut, mit einer elektronischen Auswerteeinrichtung und einer Messsonde (1), die
- einen Messkopf (6), der eine Stirnseite (76) und mindestens eine Seitenfläche (78) aufweist, und
- einen die Hautoberfläche (2) mit einem pneumatischen Druck beaufschlagenden Messsondenkanal (3) aufweist, mit dem eine Wegmessung des Deformationsweges der Hautoberfläche durchführbar ist, wobei zur Wegmessung im Bereich des Messsondenkanals (3) eine Lichtschranke (4) angeordnet ist, deren Messstrecke (7) parallel zu einer auf die Haut aufsetzbaren Öffnung (5, 23) des Messsondenkanals (3) verläuft,
**dadurch gekennzeichnet,**
**dass** die Öffnung (23) an der Seitenfläche (78) des Messkopfs (6) angeordnet ist.

9. Vorrichtung nach Anspruch 8, **dadurch gekennzeichnet, dass** die Messsonde (1) an der Öffnung (5, 23) des Messsondenkanals (3) eine die Öffnung (5, 23) umgebende Saugeinrichtung (56) aufweist.

10. Vorrichtung nach einem der Ansprüche 7 bis 9, **dadurch gekennzeichnet, dass** der Messkopf (6) auswechselbar ist.

11. Vorrichtung nach einem der Ansprüche 1 bis 10, **dadurch gekennzeichnet, dass** in der Öffnung (5, 23) ein mit Druck beaufschlagbares Kolbenelement (40) angeordnet ist.

12. Vorrichtung nach einem der Ansprüche 1 bis 11, **dadurch gekennzeichnet, dass** die Messsonde (1) an der Stelle der auf die Haut aufsetzbaren Öffnung (5, 23) abgeflacht ist.

13. Verfahren zur Messung der elastischen und viskoelastischen Verformbarkeit von Haut durch Beaufschlagung der Hautoberfläche mit einem Druck über eine Messsonde (1) und durch Messen der Hautdeformation auf Grund der Druckbelastung über eine Deformationswegmessung, wobei der Druck pneumatisch auf die zu messende Hautoberfläche über eine Öffnung (5, 23) eines Messsondenkanals (3) aufgebracht wird, und die Hautdeformation mit Hilfe einer in dem druckbeaufschlagten Messsondenkanal (3) angeordneten Lichtschranke (4) gemessen wird, deren Änderung der Lichtintensität als Maß für die Deformation der Hautoberfläche (2) verwendet wird, **dadurch gekennzeichnet, dass** die Haut im Umfeld der Austrittsöffnung (5, 23) des Messsondenkanals (3) durch Ansaugen in ihrer relativen Lage zur Messsonde (1) fixiert wird.

14. Verfahren nach Anspruch 11, **dadurch gekennzeichnet, dass** die Änderung der Lichtintensität gemessen wird, indem ein auf die Haut aufsetzbares Kolbenelement (40) gegen die Haut gedrückt wird, wobei gleichzeitig die Haut im Umfeld des in der Austrittsöffnung (5, 23) des Messsondenkanals (3) beweglichen Kolbenelementes (40) durch Ansaugen fixiert wird.
